# EUROPÄISCHE PATENTSCHRIFT

(11) **EP 4 272 791 B1**
(45) Veröffentlichungstag und Bekanntmachung des Hinweises auf die Patenterteilung: **04.06.2025**
(21) Anmeldenummer: 23193340.9
(22) Anmeldetag: 05.07.2019
(51) Int. Cl.: A61M 16/20, A61M 16/00

(54) **GASSTEUERUNGSVORRICHTUNG FÜR EIN BEATMUNGSGERÄT**
GAS CONTROL DEVICE FOR A VENTILATOR
DISPOSITIF DE COMMANDE DE GAZ POUR UN APPAREIL RESPIRATOIRE

(30) Priorität: 06.07.2018 DE 102018005341
(43) Veröffentlichungstag der Anmeldung: 08.11.2023
(62) Teilanmeldung aus: 19748647.5
(73) Patentinhaber: Löwenstein Medical Technology S.A., 2557 Luxembourg (LU)
(72) Erfinder: Adametz, Benjamin, 22609 Hamburg (DE); Gardein, Joachim, 38430 Icod de los Vinos (ES); Hahn, Henry, 22525 Hamburg (DE)
(74) Vertreter: Löwenstein Medical IP

(56) Entgegenhaltungen:
- WO-A1-2017/059667
- CN-A- 107 308 531
- DE-A1- 102016 001 140
- DE-U1- 202011 102 764
- DE-U1- 202017 005 964

## Beschreibung

Die vorliegende Offenbarung betrifft eine Gassteuerungsvorrichtung für ein Beatmungsgerät umfassend einen ersten Gaskanal mit einem Rückschlagventil, einen zweiten Gaskanal und eine Schalteinrichtung. Die Gassteuerungsvorrichtung kann in dem Beatmungsgerät angeordnet sein oder dem Beatmungsgerät zugeordnet sein. Beatmungsgeräte werden für die Therapie respiratorischer Störungen eingesetzt. Dabei können die Beatmungsgeräte in der nicht invasiven und invasiven Beatmung sowohl innerklinisch als auch außerklinisch verwendet werden.

Bei einer Beatmung eines Patienten kann in der Regel ein Beatmungsgerät mit einem inspiratorischen Zweig für den Atemgasfluss und optional mit einem Zweig für den exspiratorischen Atemgasfluss eingesetzt werden. Der Zweig für den exspiratorischen Atemgasfluss ermöglicht die Ausatmung oder Exspiration eines Atemgases durch den Patienten, während der Zweig für den inspiratorischen Atemgasfluss den Patienten mit Atemgas versorgt.

Bei den im Stand der Technik bekannten Beatmungsgeräten kann es zu Blockaden oder Störungen im exspiratorischen Zweig des Beatmungsgerätes kommen. Die nachfolgenden Dokumente sind aus dem Stand der Technik bekannt, WO 2017/059667 A1, DE 20 2017 005964 U1, CN 107 308 531 A, DE 20 2011 102764 U1 and DE 10 2016 001140 A1.

Wenn der inspiratorische Zweig des Beatmungsgerätes ein Rückschlagventil umfasst, ist eine Rückführung ausgeatmeten Atemgases in das Beatmungsgerät nicht möglich. Der Patient ist dann an einer Ausatmung gehindert.

Es ist daher Aufgabe der vorliegenden Offenbarung, eine Vorrichtung zur Verfügung zu stellen, die eine Exspiration von Atemgas auch bei einer Störung oder Blockade eines insbesondere exspiratorischen Zweiges eines Beatmungsgerätes sicherstellt.

Es ist auch Aufgabe der vorliegenden Offenbarung, eine Vorrichtung zur Verfügung zu stellen, die einen bedarfsweise aktivierbaren Gaskanal bereitstellt.

Diese erfindungsgemäße Aufgabe wird durch eine Gassteuerungsvorrichtung gemäß dem Anspruch 1 gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele.

Die vorliegende Offenbarung betrifft eine Gassteuerungsvorrichtung für ein Beatmungsgerät umfassend einen ersten Gaskanal mit einem ersten Ventil, wobei der erste Gaskanal und das Ventil eingerichtet sind, einen unter Druck stehenden Gasstrom in einer ersten Richtung passieren zu lassen und einen unter Druck stehenden Gasstrom in einer zweiten Richtung zu sperren, wobei eine Öffnung von dem ersten Gaskanal abzweigt und zu einem Umgehungskanal für das Ventil führt, wobei ein zweites Ventil eingerichtet ist, einen Gasstrom von dem ersten Gaskanal zu dem Umgehungskanal zu sperren und/oder zumindest zeitweise zu ermöglichen, wobei eine Schalteinrichtung eingerichtet ist, das zweite Ventil zu steuern, einen Gasstrom von dem ersten Gaskanal zu dem Umgehungskanal zu sperren und/oder zumindest zeitweise zu ermöglichen.

Die vorliegende Offenbarung betrifft auch eine Gassteuerungsvorrichtung umfassend einen ersten Gaskanal mit einem ersten Ventil, wobei der erste Gaskanal und das Ventil eingerichtet sind, einen unter Druck stehenden Gasstrom in einer ersten Richtung passieren zu lassen und einen unter Druck stehenden Gasstrom in einer zweiten Richtung zu sperren, wobei eine Öffnung von dem ersten Gaskanal abzweigt und zu einem Umgehungskanal für das Ventil führt, wobei ein zweites Ventil eingerichtet ist, einen Gasstrom von dem ersten Gaskanal zu dem Umgehungskanal zu sperren und/oder zumindest zeitweise zu ermöglichen, wobei eine Schalteinrichtung eingerichtet ist, das zweite Ventil zu steuern, einen Gasstrom von dem ersten Gaskanal zu dem Umgehungskanal zu sperren und/oder zumindest zeitweise zu ermöglichen.

Die vorliegende Offenbarung ist auch dadurch gekennzeichnet, dass die zweite Richtung entgegengesetzt zur ersten Richtung ist.

Die vorliegende Offenbarung ist auch dadurch gekennzeichnet, dass das erste Ventil ein pneumatisch betätigtes Rückschlagventil ist, welches durch die Kraft des unter Druck stehenden Gasstroms in der zweiten Richtung betätigt wird, sodass der Gasstrom das Rückschlagventil in einer zweiten Richtung nicht passieren kann.

Die vorliegende Offenbarung ist auch dadurch gekennzeichnet, dass der Gasstrom der zweiten Richtung Atemgas der Exspiration ist und der Gasstrom der ersten Richtung Atemgas der Inspiration ist.

Die vorliegende Offenbarung ist auch dadurch gekennzeichnet, dass eine Atemgasquelle Atemgas aus der Umgebung ansaugt und entlang des ersten Gaskanals für die Inspiration in der ersten Richtung zu dem Schlauchstutzen und dem Beatmungsschlauch befördert, wobei der Gasstrom das geöffnete Rückschlagventil passiert.

Die vorliegende Offenbarung ist auch dadurch gekennzeichnet, dass die Schalteinrichtung das Ventil öffnet und so der unter Druck stehende Gasstrom der Exspiration durch die Öffnung und durch den Umgehungskanal zumindest teilweise in die Umgebung gelangt.

Die vorliegende Offenbarung ist auch dadurch gekennzeichnet, dass das Ventil als pneumatisches Ventil ausgeführt ist, wobei eine Dichtung des Ventils die gasleitende Verbindung zwischen Öffnung und Umgehungskanal verschließt oder öffnet und wobei ein zweiter Gaskanal eingerichtet ist, einen pneumatischen Steuerdruck auf die Oberfläche der Dichtung zu leiten.

Die vorliegende Offenbarung ist auch dadurch gekennzeichnet, dass die mit dem Steuerdruck beaufschlagte Dichtung eingerichtet ist, sich dichtend an den Rand der Öffnung anzulegen und so einen Gasstrom von der Öffnung in den Umgehungskanal zu verhindern.

Die vorliegende Offenbarung ist auch dadurch gekennzeichnet, dass der zweite Gaskanal mit dem Beatmungsschlauch oder dem Gaskanal pneumatisch verbunden ist und die Schalteinrichtung die pneumatische Verbindung zumindest zeitweise öffnet oder verschließt.

Die vorliegende Offenbarung betrifft auch eine Gassteuerungsvorrichtung für ein Beatmungsgerät umfassend einen ersten Gaskanal mit einem Rückschlagventil, einen zweiten Gaskanal und eine Schalteinrichtung, wobei der erste Gaskanal eine Öffnung zum Ausströmen von Gas mit einer Dichtung und einer Verschlusskappe aufweist.

Offenbarungsgemäß ist die Gassteuerungsvorrichtung eingerichtet, bei einer Blockade oder Störung eines exspiratorischen Zweiges des Beatmungsgerätes den Gasstrom des ersten Gaskanals an dem Rückschlagventil vorbeizuführen. Dadurch wird bei einer Blockade oder Störung des exspiratorischen Zweiges eine Ausatmung des Patienten sichergestellt. Beispielsweise ist der erste Gaskanal mit dem zweiten Gaskanal verbunden. Eine getrennte Ausbildung des ersten Gaskanals und des zweiten Gaskanals, wobei der zweite Gaskanal durch einen separaten Gaskanal gespeist wird, ist in einer alternativen Ausgestaltung denkbar. Auch ist es denkbar, die offenbarungsgemäße Gassteuerungsvorrichtung ebenfalls bei einer Blockade/Störung in einem inspiratorischen Zweig des Beatmungsgerätes einzusetzen. In einem solchen Fall könnte die Gassteuerungsvorrichtung eingesetzt werden, um eine Inspiration eines Patienten bei einer Blockade oder Störung in dem inspiratorischen Zweig des Beatmungsgerätes sicherzustellen.

Die Gassteuerungsvorrichtung ist eingerichtet, bei einer Blockade bzw. Störung des exspiratorischen Zweiges den zweiten Gaskanal derart zu steuern, dass der Gasstrom des ersten Gaskanals über die Öffnung ausströmt oder an dem Rückschlagventil über einen Umgehungskanal vorbeiströmt. Der Gasstrom des ersten Gaskanals setzt sich dabei in der Regel aus einem Einatemgas und/oder einem Ausatemgas zusammen. Insbesondere bei einer Blockade oder Störung des exspiratorischen Zweiges kann das Ausatemgas eines Patienten über den inspiratorischen Zweig oder den ersten Gaskanal bis hin zu dem Rückschlagventil zurückgeführt werden. Daher ist die Öffnung des ersten Gaskanals vorteilhafterweise im Bereich des Rückschlagventils in Inspirationsflussrichtung dem Rückschlagventil nachgeschaltet angeordnet.

Die Gassteuerungsvorrichtung ist eingerichtet, den Druck in dem zweiten Gaskanal zu steuern. "Zu steuern" kann dabei bedeuten, dass die Schalteinrichtung eingerichtet sein kann, den zweiten Gaskanal freizugeben oder zu sperren. In einer alternativen Ausführungsform kann die Schalteinrichtung kontinuierlich stufenlos steuerbar sein. Basierend auf der Steuerung des Gasstroms des zweiten Gaskanals, insbesondere der Freigabe oder Sperrung des zweiten Gaskanals, kann der Gasstrom des ersten Gaskanals (das Ausatemgas oder der Ausatemgasstrom) an dem Rückschlagventil vorbeigeführt werden. Der Gasstrom des ersten Gaskanals wird über einen Umgehungskanal an dem Rückschlagventil vorbeigeführt. Der in den Umgehungskanal umgelenkte Gasstrom kann erneut dem ersten Gaskanal, vorteilhafterweise in einem Bereich, der in Inspirationsflussrichtung des Gasstroms dem Rückschlagventil vorgeschaltet liegt, zugeführt werden oder über den Umgehungskanal oder einen separaten Zweig, der als inspiratorischer oder exspiratorischer Zweig eingerichtet sein kann, abgeführt werden.

Die Gassteuerungsvorrichtung kann dabei eingerichtet sein, basierend auf einem erfassten Volumen, einem erfassten Durchfluss oder einem erfassten Druck eines Gasstroms steuerbar zu sein.

Insbesondere kann die Gassteuerungsvorrichtung mindestens einen Sensor umfassen, wobei die Schalteinrichtung eingerichtet sein kann, basierend auf einem durch den mindestens einen Sensor erfassten Parameter steuerbar zu sein.

Der mindestens eine Sensor der Gassteuerungsvorrichtung kann beispielsweise als Teil der Schalteinrichtung eingerichtet sein, mindestens einen Atmungsparameter, Atemgasparameter und/oder einen anderen Parameter aus Ausgangssignalen zu erfassen. Dabei kann ein erfasster Parameter oder können mehrere erfasste Parameter eine Funktion bilden, die sich beispielsweise auf Messungen von einem oder mehreren der folgenden Größen bezieht: (Spitzen-)Durchfluss, Durchflussrate, (Tidal-)Volumen, Druck, Temperatur, Feuchtigkeit, Geschwindigkeit, Beschleunigung, Gaszusammensetzung (z. B. eine Konzentration oder Konzentrationen von einem oder mehreren Bestandteilen), thermische Energiedissipation, (beabsichtigte) Gasleckage und/oder andere Messungen in Bezug auf den Atemgasfluss.

Atmungsparameter können beispielsweise aus Gasparametern und/oder anderen Ausgangssignalen abgeleitet werden, wobei ein oder mehrere Atmungsparameter eine oder mehrere der folgenden Größen sein können: Atmungsfrequenz, Atmungsdauer, Einatmungszeit oder -dauer, Ausatmungszeit oder -periode, Form der Atmungsflusskurve, Übergangszeit von der Einatmung zur Ausatmung und/oder umgekehrt, Übergangszeit von der (Spitzen-)Inhalationsflussrate zu der (Spitzen-)Exspirationsflussrate und/oder umgekehrt, Atmungsdruckkurvenform, maximaler proximaler Druckabfall (pro Atmungszyklus und/oder Phase), Anteil an eingeatmetem Sauerstoff und/oder andere Atmungsparameter.

Die offenbarungsgemäße Gassteuerungsvorrichtung ermöglicht es einem Patienten, auch bei einer Blockade oder Störung des exspiratorischen Zweiges des Beatmungsgerätes ausatmen zu können.

Die Gassteuerungsvorrichtung ist beispielsweise über die Schalteinrichtung eingerichtet, den Gasstrom des zweiten Gaskanals zu steuern und eine Vorbeiführung des Gasstroms des ersten Gaskanals um das Rückschlagventil durch die Öffnung zu ermöglichen. Durch die Steuerung des Gasstroms des zweiten Gaskanals kann die Öffnung, auf der die Dichtung aufliegt, die durch den Gasstrom oder Druck des zweiten Gaskanals abgedichtet wird, geöffnet werden. Der Gasstrom des ersten Gaskanals kann aus der Öffnung austreten. Bei einer Sperrung des zweiten Gaskanals ändert sich das Druckverhältnis im Bereich der Dichtung, sodass ein Gasdruck des Gasstroms des ersten Gaskanals größer ist als ein Gasdruck des zweiten Gaskanals. In der Regel ist der Gasdruck des gesperrten zweiten Gaskanals gleich null.

In der Regel ist der Umgehungskanal ringförmig um die Öffnung des ersten Gaskanals ausgebildet. Ringförmig kann auch die Öffnung umschließend bedeuten. Die ringförmige Ausgestaltung bietet den Vorteil, dass Gas in einem Winkel von bis zu 360° aus der Öffnung strömen kann. Dadurch kann eine entsprechend große Menge an exspiratorischem Ausatemgas gleichzeitig über die Öffnung des ersten Gaskanals abgeführt werden. Vorteilhafterweise ist der Umgehungskanal eingerichtet, den über die Öffnung ausgegebenen Gasstrom zu bündeln und in eine Richtung abzuführen.

In Ausgestaltung ist die Gassteuerungsvorrichtung beispielsweise über die Schalteinrichtung eingerichtet, den zweiten Gaskanal freizugeben oder zu sperren. Beispielsweise ist die Schalteinrichtung eingerichtet, den zweiten Gaskanal freizugeben, wodurch die Dichtung, die auf der Öffnung aufliegt, mit einem Gasstrom oder Druck beaufschlagt werden kann. Durch die Beaufschlagung der Dichtung mit einem Gasstrom oder Druck ist die Dichtung auf die Öffnung andrückbar. Die Schalteinrichtung kann auch eingerichtet sein, den zweiten Gaskanal zu sperren, sodass die Dichtung, die auf der Öffnung aufliegt, nicht mit einem Gasstrom oder Druck beaufschlagt werden kann. In diesem Fall liegt die Dichtung locker auf der Öffnung auf. Bei dieser Ausgestaltung kann ein Gasstrom des ersten Gaskanals durch die lose oder locker auf der Öffnung aufliegende Dichtung in einen Umgehungskanal strömen.

Vorteilhafterweise ist die Gassteuerungsvorrichtung beispielsweise über die Schalteinrichtung eingerichtet, den zweiten Gaskanal bei einer Blockade oder Störung des exspiratorischen Zweiges zu sperren. "Zu sperren" bedeutet, dass die Schalteinrichtung keinen Gasstrom oder Druck in dem zweiten Gaskanal bereitstellt oder diesen verschließt. Durch die Nichtbeaufschlagung der Dichtung mit einem Gasstrom des zweiten Gaskanals ist der Gasstrom des ersten Gaskanals ausreichend, um die Dichtung anzuheben und den Gasstrom aus dem ersten Gaskanal in den Umgehungskanal zu überführen.

Typischerweise ist die Gassteuerungsvorrichtung beispielsweise über die Schalteinrichtung eingerichtet, den zweiten Gaskanal bei einer Verwendung des Beatmungsgerätes in einem Ventilsystem freizugeben, sodass der zweite Gaskanal einen Gasstrom führen kann. Bei der Verwendung in einem Ventilsystem ist der exspiratorische oder der inspiratorische Zweig des Beatmungsgerätes oder sind der exspiratorische und der inspiratorische Zweig des Beatmungsgerätes störungsfrei und die Öffnung der Gassteuerungsvorrichtung wird durch einen Druck des zweiten Gaskanals, der in der Regel als Druckkanal ausgestaltet ist, verschlossen.

Freigeben bedeutet, dass die Gassteuerungsvorrichtung beispielsweise über die Schalteinrichtung den zweiten Gaskanal für das Durchströmen mit einem Gasstrom öffnet und somit die Dichtung, die auf der Öffnung aufliegt, mit einem Gasstrom oder Druck beaufschlagt. In der Regel wird der Gasstrom des zweiten Gaskanals mit einem Druck von 1 mbar bis 100 mbar beaufschlagt. Durch den Druck wird die Dichtung auf die Öffnung gedrückt und abgedichtet.

Die Gassteuerungsvorrichtung ist eingerichtet, bei einem Normalbetrieb des Beatmungsgerätes den Gasstrom aus dem ersten Gaskanal mittels des Gasstroms des zweiten Gaskanals, der über einen Anschluss der Verschlusskappe auf die Dichtung der Gassteuerungsvorrichtung geleitet wird, und der Dichtung abzudichten.

So kann bei einem Normalbetrieb oder einer Verwendung in einem Ventilschlauchsystem die Funktion des Rückschlagventils erhalten bleiben.

Die Gassteuerungsvorrichtung ist eingerichtet, bei einem störungsfreien Betriebszustand des Beatmungsgerätes, beispielsweise bei einem störungsfreien exspiratorischen Zweig, einen Druck über den zweiten Gaskanal auf die Dichtmembran aufzubringen und die Öffnung der Gassteuerungsvorrichtung dadurch zu verschließen.

Die Gassteuerungsvorrichtung ist eingerichtet, bei einer Blockade oder Störung des exspiratorischen Zweiges den zweiten Gaskanal zu sperren. Dadurch liegt kein Druck auf der Dichtmembran auf und die Öffnung kann durch einen Gasdruck des Gasstroms des ersten Gaskanals geöffnet werden. Der Gasstrom, der über die Öffnung austritt, kann erneut dem ersten Gaskanal zugeführt werden oder über einen separaten Zweig abführbar sein.

In einer Ausgestaltung kann die Schalteinrichtung (als Teil der Gassteuerungsvorrichtung) eingerichtet sein, basierend auf einer zeitgesteuerten Schaltung einer Exspiration und/oder einer Inspiration steuerbar zu sein.

In einer alternativen Ausgestaltung kann die Schalteinrichtung (als Teil der Gassteuerungsvorrichtung) eingerichtet sein, basierend auf einem Parameter des ersten Gaskanals steuerbar zu sein. Beispielsweise kann mittels eines Sensors mindestens ein Parameter des Gasstroms in dem ersten Gaskanal erfasst werden. Beispielsweise kann mittels eines Drucksensors ein Druck innerhalb des ersten Gaskanals erfasst werden. Die Schalteinrichtung ist dann eingerichtet, in Abhängigkeit vom erfassten Druck des Gasstroms in dem ersten Gaskanal steuerbar zu sein. Beispielsweise kann die Schalteinrichtung in der alternativen Ausgestaltung eingerichtet sein, den zweiten Gaskanal in Abhängigkeit von der Erreichung eines vorbestimmten Schwellenwertes freizugeben oder zu sperren. Ein solcher Schwellenwert kann beispielsweise ein Wert sein, der über dem durchschnittlich erfassten Druck im ersten Gaskanal liegt. Bei einer Schwellenwertüberschreitung kann die Schalteinrichtung eingerichtet sein, den zweiten Gaskanal zu sperren. Sinkt der Druck im ersten Gaskanal unter den Schwellenwert, kann die Schalteinrichtung den zweiten Gaskanal freigeben.

In einer weiteren alternativen Ausführungsform kann mittels eines Sensors beispielsweise auch die Durchflussrate oder ein Volumen des Gasstroms des ersten Gaskanals erfasst werden. Die Schalteinrichtung kann eingerichtet sein, basierend auf der ermittelten Flussrate oder dem erfassten Volumen des Gasstroms steuerbar zu sein. Auch kann die Schalteinrichtung eingerichtet sein, basierend auf weiteren Parametern des Gasstroms des ersten Gaskanals steuerbar zu sein.

In einer weiteren alternativen Ausführungsform kann die Schalteinrichtung (als Teil der Gassteuerungsvorrichtung) zudem variabel schaltbar eingerichtet sein, wobei die Schalteinrichtung eingerichtet sein kann, einen Druck des Gasstroms in dem zweiten Gaskanal zwischen 0 mbar und 1 bar, insbesondere zwischen 0 mbar und 100 mbar, stufenlos zu steuern. Die Schalteinrichtung kann auch eingerichtet sein, den Gasstrom oder Druck in dem zweiten Gaskanal kontinuierlich über einen vorbestimmten Zeitraum auf einen vorbestimmten Wert zu erhöhen.

Die Dichtung für einen Teil der Gassteuerungsvorrichtung, insbesondere für ein Ventil, ist ausgebildet, bei einem gesperrten zweiten Gaskanal einen Durchfluss des Gasstroms des ersten Gaskanals durch die Öffnung zu ermöglichen. Die Dichtung dichtet den Umgehungskanal gegen die Öffnung ab. Die Dichtung weist vorteilhafterweise eine Form auf, die die Dichtung in einem gasleeren Zustand des ersten und des zweiten Gaskanals auf der Öffnung ablegt und hält. Die Dichtung weist ein Auflageflächenverhältnis zwischen 4/5 und 2/3 auf. Die Dichtung ist derart dimensioniert, dass diese durch einen Gasstrom des ersten Gaskanals angehoben werden kann, wenn die Dichtung nicht durch einen Gasstrom des zweiten Gaskanals mit einem Gasstrom oder Druck beaufschlagt wird. Ferner umfasst die Dichtung in der Regel einen umlaufenden, verstärkten Rand, der eingerichtet ist, auf dem Rand des Umgehungskanals aufzuliegen. Der umlaufende, verstärkte Rand, kann eine Verrastungsstruktur oder Haltestruktur aufweisen, die es erleichtert, die Dichtung auf dem Rand des Umgehungskanals zu halten. Beispielsweise kann der umlaufende, verstärkte Rand eine Kante aufweisen, die an eine Hervorhebung des Randes des Umgehungskanals anliegend ausgebildet ist.

In Ausgestaltung ist die Dichtung auf der Öffnung aufliegend angeordnet und umfasst ein Gewicht. Das Gewicht, welches vorzugsweise in die Dichtung integriert ausgebildet ist, unterstützt die Positionierung der Dichtung auf der Öffnung. In der Regel weist die Dichtung in dem Bereich, in dem das Gewicht integral ausgebildet ist, im Vergleich zu den umgebenden Bereichen der Dichtung eine höhere Materialstärke auf. Die Dichtung ist typischerweise kreisförmig ausgebildet. Die Dichtung kann alternativ rechteckig, quadratisch oder oval ausgebildet sein oder eine andere geometrische Form aufweisen. Die Dichtung ist in der Regel korrespondierend zu der Form der Öffnung und/oder zu der Form des die Öffnung umgebenden Umgehungskanals ausgebildet. Zwischen dem Rand des Umgehungskanals und dem Gewicht kann die Dichtung eine Wölbung ausbilden, die in Richtung des ersten Gaskanals weist und einem Verrutschen der Dichtung vorbeugt.

Erfindungsgemäß ist das Gewicht der Dichtung ringförmig in der Mitte der Dichtung ausgebildet. In der Mitte bedeutet dabei im Zentrum der kreisförmigen Dichtung. In der Regel ist das ringförmige Gewicht unterlegscheibenförmig ausgebildet. Das Gewicht kann jedoch ebenfalls eine rechteckige oder ovale Form aufweisen. Durch die ringförmige Ausgestaltung kann die Dichtung auf einem die Öffnung umlaufend umgebenden Rand der Öffnung abgelegt werden. Die Dichtung kann auch nur bereichsweise auf dem Rand der Öffnung aufliegen. In der Regel ist die Form des Gewichts korrespondierend zur Form der Öffnung bzw. zur Form des Randes der Öffnung ausgebildet. Das Gewicht beschwert die Dichtung, wodurch die Dichtung locker auf dem Rand der Öffnung gehalten wird. Optional kann das Gewicht rechteckig ausgebildet sein oder eine andere geometrische Form aufweisen. Zudem kann das Gewicht in einem anderen Bereich der Dichtung angeordnet sein.

Erfindungsgemäß ist die Dichtung aus einem elastomeren Material ausgebildet und das Gewicht ist aus einem Metall ausgebildet. Die Dichtung ist in der Regel aus einem elastomeren Material mit einer Härte im Bereich zwischen 15 bis 25 Shore A, insbesondere zwischen 18 und 22 Shore A, ausgebildet. Beispielsweise ist die Dichtung aus einem Silikon gefertigt. Alternativ kann das Gewicht aus einem anderen Material, beispielsweise einem Hartplastik oder einer Kombination aus Metall und Kunststoff, ausgebildet sein.

In einer weiteren Weiterbildung der Offenbarung weist die Dichtung eine Ausprägung auf, die im Bereich des Gewichts in Richtung des ersten Gaskanals weisend ausgebildet ist. Die Ausprägung kann als kegelförmige Hervorhebung ausgestaltet sein, deren Spitze sich von der Öffnung in den ersten Gaskanal hineinweisend erstreckt. Beispielsweise kann die Ausprägung in der durch die unterlegscheibenförmige Ausgestaltung bedingten Ausnehmung des Gewichts ausgebildet sein. Die Ausprägung unterstützt die Dichtung auf der Öffnung des ersten Gaskanals zu halten. Die Ausprägung kann optional in einer beliebigen Form ausgebildet sein, die die Dichtung in ihrer Position auf der Öffnung hält. In einer alternativen Ausführungsform kann die Dichtung ausprägungsfrei ausgebildet sein.

In einer vorteilhaften Weiterbildung der Offenbarung ist die Verschlusskappe eingerichtet, auf die Öffnung aufsetzbar zu sein. In der Regel ist die Verschlusskappe auf die Öffnung aufklippbar. Dies bietet den Vorteil, dass die Verschlusskappe beim Aufsetzen auf die Dichtung, die auf der Öffnung aufliegt, diese in ihrer Position nicht verschiebt. Um auf die Öffnung aufklippbar zu sein, weist die Verschlusskappe in der Regel mindestens zwei Fortsätze auf, die typischerweise Widerhaken aufweisen, die in Ausnehmungen, die an einem Außenbereich der Öffnung ausgebildet sind, eingreifen. Alternativ kann die Verschlusskappe der Gassteuerungsvorrichtung mit einem Bajonettverschluss versehen sein. Optional kann die Verschlusskappe mit alternativen Verrastungselementen auf der Öffnung befestigt werden.

In einer weiteren vorteilhaften Weiterbildung der Offenbarung umfasst die Verschlusskappe einen Anschluss, der mit dem zweiten Gaskanal verbindbar ist. Die Form des Anschlusses ist in der Regel korrespondierend zu der Form des zweiten Gaskanals ausgebildet. Alternativ sind der Anschluss und der Gaskanal durch einen Adapter verbindbar. Der Anschluss ist eingerichtet, den durch die Schalteinrichtung bereitgestellten Gasstrom oder Druck des zweiten Gaskanals auf die Dichtung zu führen. Der Anschluss ist in der Regel mit der Verschlusskappe verbunden ausgebildet. Dabei ist der Anschluss typischerweise derart mit der Verschlusskappe verbunden, dass ein Gasstrom vertikal und mittig auf die Dichtung aufbringbar ist. Alternativ kann der Anschluss auch seitlich an der Verschlusskappe angeordnet sein. Der Anschluss, der ebenfalls als Kanal ausgebildet sein kann, weist beispielsweise einen Durchmesser zwischen 0,1 mm bis 0,5 mm auf.

Offenbarungsgemäß ist die Schalteinrichtung (als Teil der Gassteuerungsvorrichtung) eingerichtet, bei einem Normalbetrieb des Beatmungsgerätes den zweiten Gaskanal freizugeben und einen Gasstrom oder Druck über den zweiten Gaskanal und den Anschluss auf die Dichtung zu leiten und die Öffnung zu schließen. Bei einem Normalbetrieb des Beatmungsgerätes liegt keine Störung oder Blockade eines exspiratorischen oder inspiratorischen Zweiges des Beatmungsgerätes vor. Die Schalteinrichtung ist eingerichtet, den Gasstrom oder Druck des zweiten Gaskanals zu steuern. In der Regel wird der Gasstrom in dem zweiten Gaskanal mit einem Gasstrom oder Druck von 1 mbar bis 1 bar, insbesondere zwischen 10 mbar und 150 mbar, beaufschlagt. Dadurch wird die Dichtung auf die Öffnung des ersten Gaskanals abgedichtet, sodass der Gasstrom des ersten Gaskanals nicht über die Öffnung entweichen kann.

Offenbarungsgemäß ist die Schalteinrichtung (als Teil der Gassteuerungsvorrichtung) eingerichtet, bei einer Blockade oder Störung des exspiratorischen Zweiges den zweiten Gaskanal zu sperren. Durch die Sperrung des zweiten Gaskanals kommt der Gasstrom oder Druck des zweiten Gaskanals zum Erliegen. Die Gassteuerungsvorrichtung kann den Gasstrom des ersten Gaskanals über die Öffnung ausleiten. Bei einer Sperrung des zweiten Gaskanals durch die Schalteinrichtung ist der Gasstrom oder Druck im ersten Gaskanal größer als im zweiten Gaskanal. Dadurch kann der Gasstrom des ersten Gaskanals die Dichtung, die auf der Öffnung aufliegt, anheben und den Gasstrom in den Umgehungskanal führen und das Rückschlagventil umgehen. Die Dichtung wird derart angehoben, dass die Dichtung im Bereich der Begrenzung der Dichtung durch die Verschlusskappe an den Rand des Umgehungskanals angedrückt oder gehalten wird. Sobald der zweite Gaskanal gesperrt ist und kein Gasstrom oder Druck mehr auf die Dichtung aufgebracht wird, wird das Gewicht der Dichtung durch den Gasstrom des ersten Gaskanals angehoben, sodass die Dichtung, die auf dem Rand der Öffnung aufliegt, angehoben wird, wodurch der Gasstrom von dem ersten Gaskanal über die Öffnung in den Umgehungskanal strömen kann.

Die vorliegende Offenbarung umfasst ferner auch eine Dichtung für einen Teil der Gassteuerungsvorrichtung für ein Beatmungsgerät umfassend einen ersten Gaskanal mit einem Rückschlagventil, einen zweiten Gaskanal und eine Schalteinrichtung, wobei der erste Gaskanal eine Öffnung zum Ausströmen von Gas mit einer Dichtung und einer Verschlusskappe aufweist.

Offenbarungsgemäß weist die Dichtung ein Gewicht auf und ist eingerichtet, bei einem gesperrten zweiten Gaskanal einem Gasstrom des ersten Gaskanals einen Durchlass durch die Öffnung zu ermöglichen. Die Dichtung ist in der Regel kreisförmig ausgebildet. Alternativ kann die Dichtung jedoch auch rechteckig ausgebildet sein oder eine andere geometrische Form aufweisen. Typischerweise ist die Dichtung aus einem elastomeren Material, beispielsweise Silikon, ausgebildet. Die Dichtung weist in der Regel eine Härte zwischen 15 bis 25 Shore A, insbesondere zwischen 18 und 22 Shore A auf. Das Gewicht kann in einer alternativen Ausgestaltung in einem anderen Bereich der Dichtung ausgebildet sein.

In Ausgestaltung weist die Dichtung einen zumindest teilweise umlaufenden Rand auf, der eingerichtet ist, in eine Ausnehmung der Gassteuerungsvorrichtung einzugreifen. Der umlaufende Rand ist vorzugsweise verstärkt ausgebildet und kann eine Ausprägung aufweisen, die in eine Ausnehmung am Rand des Umgehungskanals eingreift. Der umlaufende Rand der Dichtung dient zum einen der Abdichtung und zum anderen der Positionierung der Dichtung auf dem Rand des Umgehungskanals. Er wird durch die Verschlusskappe auf den Rand des Umgehungskanals gedrückt oder gehalten oder abgedichtet. Der Rand des Umgehungskanals kann eine Hervorhebung aufweisen, die eingerichtet ist, an die Dichtung derart anliegend zu sein, dass eine Kante der Dichtung mit der Hervorhebung in Eingriff kommt.

Die vorliegende Offenbarung betrifft auch eine Gassteuerungsvorrichtung, die als elektrische und/oder pneumatische Steuereinheit in einem Beatmungsgerät ausgebildet und eingerichtet ist, einen unter Druck stehenden Gasstrom in einer ersten Richtung passieren zu lassen und einen unter Druck stehenden Gasstrom in einer zweiten Richtung zu sperren und einen Gasstrom von dem ersten Gaskanal zu dem Umgehungskanal zu sperren und/oder zumindest zeitweise zu ermöglichen, wobei die Gassteuerungsvorrichtung eingerichtet ist, einen Gasstrom von dem ersten Gaskanal zu dem Umgehungskanal zu sperren und/oder zumindest zeitweise zu ermöglichen.

Die Gassteuerungsvorrichtung kann als elektrische und/oder pneumatische Steuereinheit ausgebildet sein, die unterschiedliche Aktoren und Sensoren umfasst, beispielsweise zumindest die Schalteinrichtungen, Ventile und die Gasquelle, und diese koordiniert im Sinne der Offenbarung ansteuert.

Die Gassteuerungsvorrichtung kann als elektrische und/oder pneumatische Steuereinheit ausgebildet und eingerichtet sein, einen unter Druck stehenden Gasstrom in einer ersten Richtung passieren zu lassen und einen unter Druck stehenden Gasstrom in einer zweiten Richtung zu sperren und einen Gasstrom von dem ersten Gaskanal zu dem Umgehungskanal zu sperren und/oder zumindest zeitweise zu ermöglichen, wobei eine Gassteuerungsvorrichtung eingerichtet ist, einen Gasstrom von dem ersten Gaskanal zu dem Umgehungskanal zu sperren und/oder zumindest zeitweise zu ermöglichen.

Die vorliegende Offenbarung umfasst des Weiteren eine Verschlusskappe für eine Gassteuerungsvorrichtung für ein Beatmungsgerät umfassend einen ersten Gaskanal mit einem Rückschlagventil, einen zweiten Gaskanal und eine Schalteinrichtung, wobei der erste Gaskanal eine Öffnung zum Ausströmen von Gas mit einer Dichtung und einer Verschlusskappe aufweist.

Offenbarungsgemäß weist die Verschlusskappe zumindest bereichsweise Fortsätze auf, die Widerhaken ausbilden und eingerichtet sind, in Ausnehmungen an der Gassteuerungsvorrichtung einzugreifen. Alternativ kann die Verschlusskappe einen Bajonettverschluss umfassen, der eingerichtet ist, in Ausnehmungen an der Öffnung einzugreifen.

Die vorliegende Offenbarung umfasst des Weiteren ein Beatmungsgerät umfassend eine Gassteuerungsvorrichtung gemäß mindestens einem der vorgenannten Merkmale.

Gas kann im Sinne der Offenbarung jedes atembare Gas oder Gasgemisch sein, insbesondere Luft, Sauerstoff, Atemgas der Exspiration oder Atemgas der Inspiration. Im Folgenden werden anhand von stark vereinfachten schematischen Darstellungen bevorzugte Ausführungsbeispiele der Offenbarung näher erläutert. Es zeigt:
- Fig. 1a: eine schematische Ansicht einer offenbarungsgemäßen Gassteuerungsvorrichtung,
- Fig. 1b: eine schematische Ansicht einer offenbarungsgemäßen Gassteuerungsvorrichtung, wobei die Gassteuerungsvorrichtung hier nur teilweise dargestellt ist, nämlich als Schalteinrichtung oder als Ventilblock,
- Fig. 2: eine schematische Explosionsansicht der in Figur 1b gezeigten Gassteuerungsvorrichtung,
- Fig. 3: einen Längsschnitt durch die in den Figuren 1b und 2 gezeigte Gassteuerungsvorrichtung,
- Fig. 4a: eine Seitenansicht der in den Figuren 1b bis 3 gezeigten Gassteuerungsvorrichtung,
- Fig. 4b: eine Draufsicht auf eine erfindungsgemäße Verschlusskappe der in den Figuren 1b bis 4a gezeigten Gassteuerungsvorrichtung,
- Fig. 4c: eine Seitenansicht auf ein Rückschlagventil der in den Figuren 1b bis 4b gezeigten Gassteuerungsvorrichtung.

In den Figuren weisen dieselben konstruktiven Elemente jeweils dieselben Bezugsziffern auf.

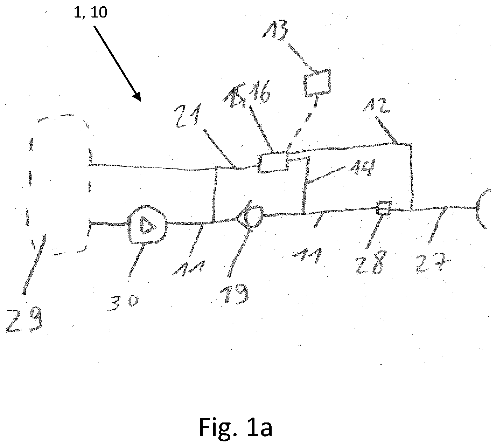

Figur 1a zeigt eine schematische Ansicht einer offenbarungsgemäßen Gassteuerungsvorrichtung 10 für ein Beatmungsgerät 1. Beatmungsgeräte 1 weisen in der Regel einen inspiratorischen Zweig, der für die Zuführung von Atemgas zu einem Patienten vorgesehen ist, und einen exspiratorischen Zweig auf, der zur Abführung von durch den Patienten ausgeatmetem Atemgas dient. Die Gassteuerungsvorrichtung 10 kann als elektrische und/oder pneumatische Steuereinheit ausgebildet und eingerichtet sein, einen unter Druck stehenden Gasstrom in einer ersten Richtung passieren zu lassen und einen unter Druck stehenden Gasstrom in einer zweiten Richtung zu sperren und einen Gasstrom von dem ersten Gaskanal 11 zu dem Umgehungskanal 21 zu sperren und/oder zumindest zeitweise zu ermöglichen, wobei eine Gassteuerungsvorrichtung 10 eingerichtet ist, einen Gasstrom von dem ersten Gaskanal 11 zu dem Umgehungskanal 21 zu sperren und/oder zumindest zeitweise zu ermöglichen.

Die in der Figur 1a gezeigte offenbarungsgemäßen Gassteuerungsvorrichtung 10 ist zur Verwendung in einem Beatmungsgerät 1 oder als Teil eines Beatmungsgerätes vorgesehen und weist einen ersten Gaskanal 11 mit einem ersten Ventil 19 auf, wobei der erste Gaskanal 11 und das Ventil 19 eingerichtet sind, einen unter Druck stehenden Gasstrom in einer ersten Richtung passieren zu lassen und einen unter Druck stehenden Gasstrom in einer zweiten Richtung zu sperren, wobei eine Öffnung 14 von dem ersten Gaskanal 11 abzweigt und zu einem Umgehungskanal 21 für das Ventil 19 führt, wobei ein zweites Ventil 15, 16 eingerichtet ist, einen Gasstrom von dem ersten Gaskanal 11 zu dem Umgehungskanal 21 zu sperren und/oder zumindest zeitweise zu ermöglichen, wobei eine Schalteinrichtung 13 eingerichtet ist, das zweite Ventil 15, 16 zu steuern, einen Gasstrom von dem ersten Gaskanal 11 zu dem Umgehungskanal 21 zu sperren und/oder zumindest zeitweise zu ermöglichen.

Die Schalteinrichtung umfasst hier beispielsweise den Ventilblock 13 mit dem ersten Ventil 19, dem zweiten Ventil 15, 16, dem ersten Gasweg 11 und dem zweiten Gasweg 12 und der Umgehung 14, 21. Optional umfasst die Schalteinrichtung 13 auch die pneumatische oder elektronische Steuerung für die Komponenten des Ventilblocks. Eine Gas- oder Atemgasquelle 30, beispielsweise ein Gebläse, befördert Gas oder Atemgas, beispielsweise angesaugt aus der Umgebung 29, entlang des ersten Gaskanals 11 für die Inspiration in der ersten Richtung zu dem Schlauchstutzen 28 und dem Beatmungsschlauch 27. Dabei passiert der Gasstrom das geöffnete Rückschlagventil 19.

Eine Exspiration des Patienten sorgt für einen Atemgasstrom in einer zweiten Richtung durch den Beatmungsschlauch, den Stutzen 28 und den ersten Gaskanal 11 bis zu dem Rückschlagventil 19. Dieses ist ein pneumatisch betätigtes Rückschlagventil 19, welches durch die Kraft des unter Druck stehenden Gasstroms der Exspiration in der zweiten Richtung betätigt wird und so das Rückschlagventil 19 nicht passieren kann. Da die Öffnung 14 mit dem ersten Gaskanal 11 in Verbindung steht, breitet sich der unter Druck stehende Gasstrom der Exspiration in der zweiten Richtung auch in die Öffnung aus und gelangt bis zu dem Ventil 15, 16. Sofern die Schalteinrichtung 13 das Ventil 15, 16 öffnet, strömt der unter Druck stehende Gasstrom der Exspiration weiter entlang der Öffnung und des Umgehungskanals 21 in die Umgebung 29 oder alternativ in den ersten Gaskanal und von dort zumindest teilweise in die Umgebung.

Das Ventil 15, 16 kann als pneumatisches Ventil ausgeführt sein. In diesem Fall kann eine Dichtung 15 des Ventils die gasleitende Verbindung zwischen Öffnung 14 und Umgehungskanal verschließen oder öffnen. Der Steuerimpuls für die Dichtung 15 des Ventils kommt von einem zweiten Gaskanal 12, der einen pneumatischen Steuerdruck auf die Oberfläche der Dichtung 15 leitet. Ist die Dichtung mit dem Steuerdruck beaufschlagt, so legt sie sich dichtend an den Rand 22 der Öffnung 14 und verhindert so einen Gasstrom von der Öffnung in den Umgehungskanal.

Der zweite Gaskanal kann dazu aus dem Beatmungsschlauch oder dem Gaskanal 11 pneumatisch gespeist werden. Ebenfalls ist eine andere Druckgasquelle, wie ein Steuergebläse, denkbar. Die Schalteinrichtung 13 öffnet oder verschließt die pneumatische Verbindung zwischen dem zweiten Gaskanal 12 und dem Beatmungsschlauch oder dem Gaskanal 11 zumindest zeitweise.

Das Ventil 15, 16 kann als Schaltventil ausgeführt sein. In diesem Fall öffnet oder verschließt eine Aktivierung des Ventils durch die Schalteinrichtung 13 den Gasstrom von der Öffnung 14 in den Umgehungskanal. In diesem Fall ist ein zweiter Gaskanal entbehrlich.

Beatmungsgeräte weisen in der Regel einen inspiratorischen Zweig, der für die Zuführung von Atemgas zu einem Patienten vorgesehen ist, und einen exspiratorischen Zweig auf, der zur Abführung von durch den Patienten ausgeatmetem Atemgas dient.

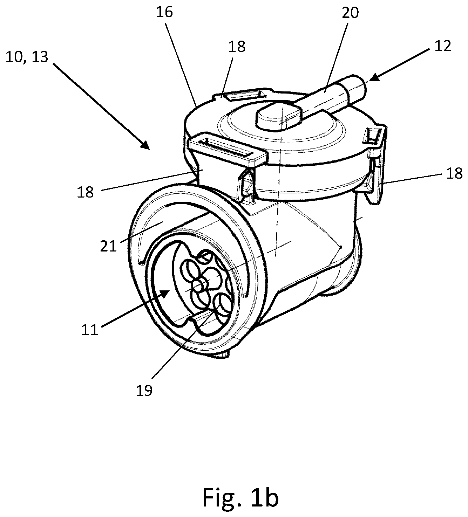

Figur 1b zeigt eine schematische Ansicht einer erfindungsgemäßen Gassteuerungsvorrichtung 10 für ein Beatmungsgerät. Die Gassteuerungsvorrichtung 10 ist hier nur teilweise dargestellt, nämlich als Schalteinrichtung 13 oder als Ventilblock, und umfasst einen ersten Gaskanal 11 mit einem ersten Ventil 19, wobei der erste Gaskanal 11 und das Ventil 19 eingerichtet sind, einen unter Druck stehenden Gasstrom in einer ersten Richtung passieren zu lassen und einen unter Druck stehenden Gasstrom in einer zweiten Richtung zu sperren, wobei eine Öffnung 14 von dem ersten Gaskanal 11 abzweigt und zu einem Umgehungskanal 21 für das Ventil 19 führt, und ein zweites Ventil 15, 16, um den Umgehungskanal 21 zu öffnen oder zu schließen.

Die in der Figur 1 und 1b gezeigte offenbarungsgemäße Gassteuerungsvorrichtung 10 ist zur

Verwendung in einem Beatmungsgerät vorgesehen und weist einen ersten Gaskanal 11 (inspiratorischen Zweig) auf, der einen Gasstrom in Richtung eines Patienten führt. Der erste Gaskanal 11 umfasst ein Rückschlagventil 19, sowie eine Öffnung mit einer Dichtung. Die Öffnung und die Dichtung sind in der Figur 1 von einer Verschlusskappe 16 verdeckt. Das Rückschlagventil 19 verhindert eine Rückführung eines von dem Patienten kommenden, durch die Ausatmung verunreinigten Atemgases oder Gasstroms.

Zudem weist die Gassteuerungsvorrichtung 10 einen zweiten Gaskanal 12 auf, der einen Gasstrom zu der Öffnung führt. Der zweite Gaskanal 12 ist über einen Anschluss 20 mit der Verschlusskappe 16 verbunden, die auf die Öffnung des ersten Gaskanals aufgeklippt ist. Der zweite Gaskanal 12 verläuft über den Anschluss 20 zu der Verschlusskappe 16 und wird durch eine Schalteinrichtung gesteuert. Der zweite Gaskanal 12 kann von einem Gasstrom durchflossen werden.

Die Schalteinrichtung kann den zweiten Gaskanal 12 freigeben oder sperren. Dabei gibt die Schalteinrichtung den zweiten Gaskanal 12 für einen Gasstrom oder einen Gasdruck oder ein Gasstromvolumen frei. Bei einem Normalbetrieb des Beatmungsgerätes ist der zweite Gaskanal 12 freigegeben, sodass der Gasstrom oder Druck über den Anschluss 20 auf die Dichtung innerhalb der Verschlusskappe 16 führbar ist. Durch den Gasstrom oder Druck des zweiten Gaskanals 12 wird die Dichtung vollständig auf die Öffnung gedrückt und die Öffnung somit verschlossen. Dadurch wird bei einem Normalbetrieb des Beatmungsgerätes, bei dem keine Blockade vorliegt, ein Ausströmen eines inspiratorischen Gasstroms des ersten Gaskanals 11, der zu dem Patienten geführt werden soll, über die Öffnung in den Umgehungskanal 21 verhindert. Dies ist in diesem Fall nicht notwendig, da der exspiratorische Zweig blockadefrei ist, sodass das ausgeatmete Atemgas des Patienten über den exspiratorischen Zweig abgegeben werden kann.

Bei einer Blockade oder Störung eines exspiratorischen Zweiges des Beatmungsgerätes wird hingegen ein Ausatmen des Patienten verhindert oder erschwert. Durch das Rückschlagventil 19 kann der Patient zudem nicht über den inspiratorischen Zweig ausatmen. Bei einer Blockade oder Störung des exspiratorischen Zweiges des Beatmungsgerätes sperrt die Schalteinrichtung den zweiten Gaskanal 12. Durch den gesperrten Gaskanal 12 kommt der Gasstrom des zweiten Gaskanals 12, der auf die Dichtung wirkt, zum Erliegen, wodurch der Gasstrom des ersten Gaskanals 11 die Dichtung, die auf der Öffnung aufliegt, anheben kann, sodass der Gasstrom des ersten Gaskanals über einen Umgehungskanal 21 um das Rückschlagventil herumgeführt wird. In diesem Fall ist somit der Gasstrom oder Druck des ersten Gaskanals 11 größer als der Gasstrom oder Druck des zweiten Gaskanals 12, wodurch der Gasstrom des ersten Gaskanals 11 die Dichtung, die auf der Öffnung aufliegt, anheben kann. Die Dichtung ist derart dimensioniert und ausgebildet, dass der Gasstrom des ersten Gaskanals 11 bei einem gesperrten Gaskanal 12 diese anheben kann. Anheben bedeutet dabei, dass die Dichtung an ihrem umlaufenden Rand durch die Verschlusskappe 16 auf dem Rand des Umgehungskanals 21 umlaufend gehalten wird und in Richtung des Zentrums der kreisförmig ausgestalteten Dichtung durch den Gasstrom des ersten Gaskanals 11 hochgedrückt wird. Das Gewicht der Dichtung ist derart proportioniert, dass es die Dichtung bei einem Nachlassen des Gasstroms des zweiten Gaskanals 12 automatisch auf der Öffnung ablegt.

Der Patient kann somit auch bei einer Blockade oder Störung des exspiratorischen Zweiges ausatmen.

Bei einem Leckagesystem sperrt die Schalteinrichtung den zweiten Gaskanal bzw. gibt keinen Druck auf die Dichtung. In diesem Fall ist das Schaltventil eingerichtet, dauerhaft ein Ausströmen von Gas zu ermöglichen.

Bei einem Ventilsystem ist die Schalteinrichtung eingerichtet, entsprechend der zeitgesteuerten Exspiration des Beatmungsgerätes zu schalten. Somit ist die Schalteinrichtung eingerichtet, bei der Schaltung des Beatmungsgerätes auf eine Exspiration den zweiten Gaskanal 12 zu sperren. Dadurch wird die Exspiration eines Patienten sichergestellt, da die Öffnung der Gassteuerungsvorrichtung während der Exspiration geöffnet ist und rückströmendes Gas über die Öffnung entweichen kann.

Bei einem Normalbetrieb des exspiratorischen Zweiges des Beatmungsgerätes schaltet die Schalteinrichtung den zweiten Gaskanal 12 frei, wodurch ein Gasstrom in den zweiten Gaskanal 12 strömen kann und mit einem Druck beaufschlagt wird. Durch den Gasstrom in dem zweiten Gaskanal 12 wird die Dichtung auf der Öffnung abgedichtet, wodurch ein Ausströmen von inspiratorischem Gas durch die Öffnung während des Normalbetriebs verhindert wird.

Die Verschlusskappe 16 umfasst in der vorliegenden Ausführungsform drei Fortsätze 18, mittels derer die Verschlusskappe 16 auf die Öffnung des ersten Gaskanals 11 aufklippbar ist. Zudem ist an der Verschlusskappe ein Anschluss 20 ausgebildet, mit dem der zweite Gaskanal 12 verbindbar ist. Der Anschluss 20 ist mittig auf der Verschlusskappe 16 angeordnet, sodass der Gasstrom oder Druck über den Anschluss 20 und die Verschlusskappe 16 mittig und gleichmäßig auf die Dichtung aufbringbar ist.

Die in der Figur 1b gezeigte Gassteuerungsvorrichtung 10 ist somit bei einem Normalbetrieb eingerichtet, den Gasstrom in dem ersten Gaskanal 11 durch das Rückschlagventil 19 zu dem Patienten hinzuführen. Bei einem Normalbetrieb des exspiratorischen Zweiges wird die Öffnung des ersten Gaskanals 11 verschlossen und die Funktion des Rückschlagventils 19 bleibt bestehen. Bei einer Blockade oder Störung des Rückschlagventils 19 umgeht die Gassteuerungsvorrichtung 10 das Rückschlagventil 19.

Die Schalteinrichtung der Gassteuerungsvorrichtung 10 wird durch die zeitgesteuerte Schaltung des Beatmungsgerätes zwischen Inspiration und Exspiration getriggert. In der Regel sperrt die Schalteinrichtung den zweiten Gaskanal 12, sobald das Beatmungsgerät auf Exspiration umschaltet. Wahlweise kann die Schaltung zeitverzögert erfolgen. In der Regel gibt die Schalteinrichtung den zweiten Gaskanal 12 frei, wenn das Beatmungsgerät auf Inspiration schaltet.

In der Figur 1b ist ferner der Umgehungskanal 21 dargestellt. Der Umgehungskanal 21 erstreckt sich von der Öffnung und ermöglicht die Vorbeiführung des aus der Öffnung ausströmenden Gasstroms an dem Rückschlagventil 19 vorbei. Der Umgehungskanal 21 ist umlaufend um die Öffnung ausgebildet und verengt sich in seinem weiteren Verlauf zu einem Kanal, der sich in einer Richtung parallel zu dem ersten Gaskanal erstreckt. Der Umgehungskanal 21 kann anschließend erneut in den ersten Gaskanal einmünden oder als separater Zweig den an dem Rückschlagventil vorbeigeführten Gasstrom abführen.

Der Umgehungskanal weist im Bereich der Öffnung einen Rand auf, den die Dichtung durch die Verschlusskappe 16 abdichten kann. Der Rand des Umgehungskanals 21 kann eine Hervorhebung aufweisen, die in eine Kante der Dichtung eingreifen kann. Der Rand des Umgehungskanals 21 verhindert ein Verrutschen der Dichtung.

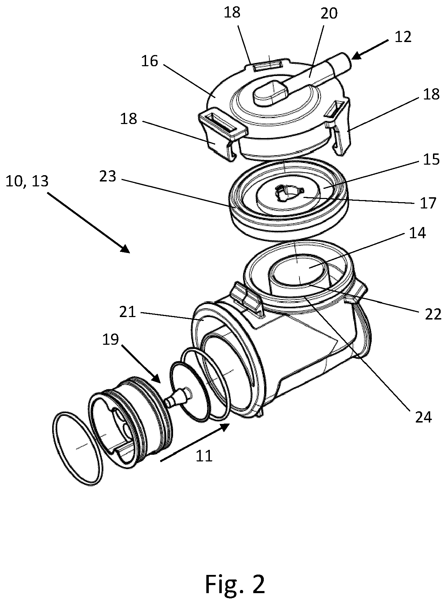

Figur 2 zeigt eine schematische Explosionsansicht der in Figur 1 gezeigten Gassteuerungsvorrichtung 10. Dabei sind der erste und der zweite Gaskanal 11, 12, das Rückschlagventil 19 sowie die Öffnung 14 mit der Dichtung 15 gezeigt.

Dargestellt ist auch die Verschlusskappe 16 mit dem Anschluss 20. Gezeigt ist die mittige Anordnung des Anschlusses 20, wobei ein Ende des Anschlusses 20 sich nach außen erstreckt und mit dem zweiten Gaskanal 12 verbindbar ist.

In der Figur 2 ist gezeigt, dass die Öffnung 14 einen Rand 22 aufweist. Der Rand 22 ist als Erhebung ausgebildet und dient als Ablage für das in die Dichtung 15 integriert ausgebildete Gewicht 17.

Die Dichtung 15 ist aus einem elastomeren Material, vorzugsweise einem Silikon mit einer Härte zwischen 15 bis 25 Shore A, insbesondere zwischen 18 und 22 Shore A, ausgebildet. Die Dichtung 15 ist kreisförmig ausgebildet und weist eine Begrenzung 23 auf.

Die Begrenzung 23 kann als eine verstärkte Struktur oder als funktionale Hervorhebung ausgebildet sein. Die Begrenzung 23 ist eingerichtet, auf einem Rand 24 des Umgehungskanals 21 aufzuliegen. Die Dichtung 15 kann zwischen der Begrenzung 23 der Dichtung 15 und dem Gewicht 17 eine dünnere Materialstärke aufweisen als im Bereich des Randes 24 des Umgehungskanals 21 und des Bereichs der Dichtung 15, in dem das Gewicht 17, zumeist integral, angeordnet ist.

In der vorliegenden Ausführungsform weist die Dichtung 15 zwischen dem Rand 24 des Umgehungskanals 21 und dem Gewicht 17 eine gewölbte Struktur auf, die zusätzlich einer Verschiebung der Dichtung 15 auf der Öffnung 14 vorbeugen kann. In weiteren Ausführungsformen kann die Dichtung 15 zwischen dem Rand 24 des Umgehungskanals 21 und dem Gewicht 17 eine andere Struktur aufweisen oder strukturfrei ausgebildet sein.

Das Gewicht 17 der Dichtung 15 ist in Form einer Unterlegscheibe ausgebildet. Das Gewicht 17 ist aus einem Metall gebildet und in die Dichtung 17 integral ausgebildet. Das Gewicht 17 stabilisiert die Dichtung 15 auf der Öffnung 14.

Beim Aufsetzen der Verschlusskappe 16 auf die Öffnung 14 wird die Dichtung 15 im Bereich der Begrenzung 23 der Dichtung 15 an den Rand des Umgehungskanals 21 angedrückt und gehalten. Die Verschlusskappe 16 dichtet die Dichtung 15 nach außen ab und hält die Dichtung 15 in ihrer Position innerhalb der Verschlusskappe 16. Die Dichtung 15 wird somit innerhalb der Gassteuerungsvorrichtung 10 durch die aufgeklippte Verschlusskappe 16 auf dem Rand 24 des Umgehungskanals 21 angedrückt oder gehalten. Die Dichtung 15 wird über die umlaufend ausgebildete, verstärkte Begrenzung 23 zwischen dem Rand 24 des Umgehungskanals 21 und der Verschlusskappe 16 eingeklemmt oder abgedichtet.

Bereiche der Dichtung 15, die sich von der Begrenzung 23 unterscheiden,
sind berührungsfrei zu der Verschlusskappe 16 ausgebildet. Diese Bereiche können auch als Beaufschlagungsbereiche bezeichnet werden, da bei einer Freigabe des zweiten Gaskanals 12 durch die Schalteinrichtung diese Bereiche der Dichtung 15 mit einem Gasstrom oder Druck beaufschlagt werden.

Die Dichtung 15 wird in einem Ruhezustand (ohne Gasströme) durch das Gewicht und ihre Struktur auf der Öffnung 14 gehalten. Ist die Schalteinrichtung eingerichtet, den zweiten Gaskanal 12 freizugeben, werden zusätzlich die Beaufschlagungsbereiche der Dichtung 15 mit einem Gasstrom oder Druck beaufschlagt, wodurch die Dichtung 15 die Öffnung 14 abdichtet.

Ist die Schalteinrichtung eingerichtet, den zweiten Gaskanal 12 zu sperren, werden die Beaufschlagungsbereiche der Dichtung 15 nicht mit einem Gasstrom beaufschlagt, sodass der Gasstrom des ersten Gaskanals 11 ausreichend ist, um die Dichtung im Bereich der Beaufschlagungsbereiche anzuheben und den Gasstrom in den Umgehungskanal 21 zu führen.

Die in der Figur 2 gezeigte Verschlusskappe 16 umfasst Fortsätze 18, die Widerhaken ausbilden. Die Widerhaken greifen beim Aufklippen der Verschlusskappe 16 in Ausnehmungen ein, die an der Außenseite der Gassteuerungsvorrichtung 10 ausgebildet sind. In weiteren Ausführungsformen sind weitere Verschlussmöglichkeiten, beispielsweise eine Verrastung in Form eines Bajonettverschlusses, denkbar. Ferner sind in der Figur 2 das Rückschlagventil 19 sowie der Umgehungskanal 21 dargestellt.

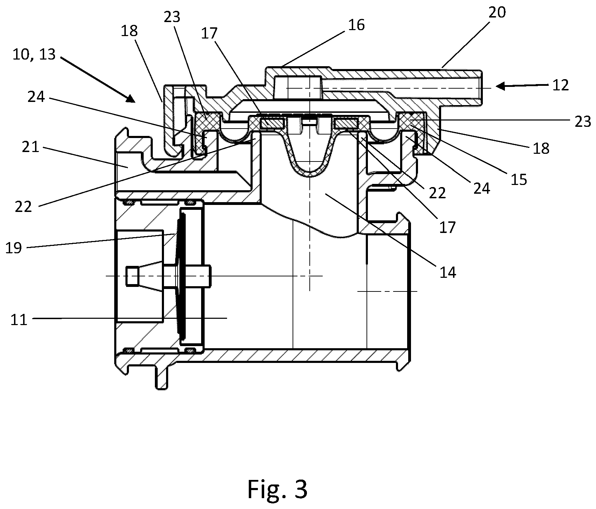

Figur 3 zeigt einen Längsschnitt durch die in den Figuren 1 und 2 gezeigte Gassteuerungsvorrichtung 10. Dargestellt ist der erste Gaskanal 11 mit dem Rückschlagventil 19, der Öffnung 14 und der Dichtung 15 sowie der zweite Gaskanal 12. Zudem sind die Verschlusskappe 16 mit dem Anschluss 20 und der Umgehungskanal 21 dargestellt.

Über den ersten Gaskanal 11 kann ein inspiratorischer Gasstrom zu dem Patienten gefördert werden. Das Rückschlagventil 19 verhindert ein Rückströmen von Ausatemgas in das Beatmungsgerät über den ersten Gaskanal 11. Bei einer Blockade oder Störung des exspiratorischen Zweiges des Beatmungsgerätes kann somit über den ersten Gaskanal 11 keine Exspiration des Gasstroms oder Ausatemgases erfolgen.

Gezeigt ist zudem der Umgehungskanal 21, der sich von der Öffnung 14 aus erstreckt. Der Umgehungskanal 21 ist als umlaufender Kanal um die Öffnung 14 ausgebildet und erstreckt sich in seinem weiteren Verlauf zu einem Kanal, der sich in einer Richtung parallel zu dem ersten Gaskanal 11 erstreckt. Durch die umlaufende Anordnung des Umgehungskanals kann eine große Menge an Ausatemgas gleichzeitig um das Rückschlagventil 19 vorbei und über die Öffnung 14 abgeführt werden. Der Umgehungskanal 21 kann in den inspiratorischen Zweig einmünden oder als separater exspiratorischer Zweig ausgebildet sein.

Die Dichtung 15 ist kreisförmig ausgebildet und weist in ihrem Zentrum ein Gewicht 17 auf. Das Gewicht 17 ist ringförmig oder unterlegscheibenförmig ausgebildet. Das Gewicht 17 weist in seiner formbedingten Aussparung eine kegelförmige Ausgestaltung der Dichtung 15 auf, die sich in Richtung des ersten Gaskanals 11 erstreckt. Die kegelförmige Ausgestaltung bietet den Vorteil, dass die Dichtung 15 in ihrer Position auf der Öffnung 14 gegen Verrutschen gesichert ist. In weiteren Ausführungsformen kann die Ausgestaltung der Dichtung 15 eine andere geometrische Form aufweisen, die geeignet ist, die Dichtung 15 in ihrer Position zu halten. Das Gewicht 17 ist in die Dichtung 15 integriert ausgebildet. Die Dichtung 15 weist im Bereich des Gewichts 17 eine größere Materialstärke auf. Dies bietet zum einen den Vorteil, dass das Gewicht 17 in die Dichtung 15 integrierbar ist, und zum anderen den Vorteil, dass durch die höhere Materialstärke ein zusätzliches Gewicht 17 erzeugt wird, das die Dichtung 15 in ihrer Position hält. Das Gewicht 17 kann auch auf die Dichtung 15 aufgebracht ausgebildet sein.

Die in der Figur 3 gezeigte Verschlusskappe 16 ist auf der Öffnung 14 angeordnet, wobei die Verschlusskappe 16 im Bereich der Begrenzung 23 die Dichtung 15 auf den Rand des Umgehungskanals 21 drückt, während die Dichtung 15 in den Beaufschlagungsbereichen berührungsfrei zu der Verschlusskappe 16 angeordnet ist.

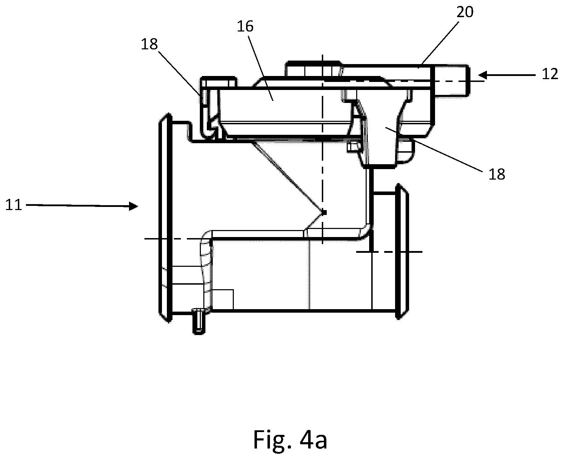

Figur 4a zeigt eine Seitenansicht der in den Figuren 1 bis 3 gezeigten Gassteuerungsvorrichtung 10. Dargestellt ist der erste Gaskanal 11 sowie die Verschlusskappe 16 mit den Fortsätzen 18 und dem Anschluss 20 für den zweiten Gaskanal.

In der Figur 4a ist zu erkennen, dass die Eingangsseite des ersten Gaskanals 11 größer dimensioniert ist als die Ausgangsseite. Die Eingangsseite umfasst neben dem ersten Gaskanal 11 auch den zu einem Kanal verengten Umgehungskanal 21. Somit umfasst die Eingangsseite des ersten Gaskanals 11 sowohl den in Inspirationsflussrichtung ausgerichteten ersten Gaskanal 11 sowie den entgegen der Inspirationsflussrichtung ausgerichteten Umgehungskanal 21.

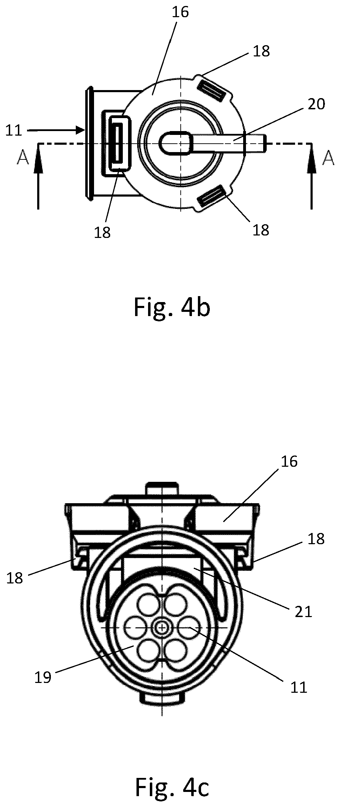

Figur 4b zeigt eine Draufsicht auf die offenbarungsgemäße Verschlusskappe 16 der in den Figuren 1 bis 4b gezeigten erfindungsgemäßen Gassteuerungsvorrichtung 10. Die Verschlusskappe 16 ist auf die Öffnung des ersten Gaskanals aufklippbar. Beim Aufklippen der Verschlusskappe 16 wird die Dichtung im Bereich des Gewichts von der Verschlusskappe 16 auf eine - in Figur 3 gezeigte - Erhebung 22 der Öffnung gedrückt und abgedichtet.

Die Verschlusskappe 16 weist einen Anschluss 20 für den zweiten Gaskanal 12 auf. Über den Anschluss 20 kann der Gasstrom des zweiten Gaskanals 12 auf die Dichtung aufgebracht werden, um die Öffnung zu verschließen. Die Verschlusskappe 16 umfasst ferner die Fortsätze 18, die zumeist Widerhaken ausbilden und eingerichtet sind, in Ausnehmungen im Bereich der Öffnung einzugreifen. In weiteren Ausführungsformen kann die Verschlusskappe 16 weitere Fortsätze 18 aufweisen oder über ähnliche Verrastungselemente auf der Öffnung befestigbar sein.

Figur 4c zeigt eine Draufsicht auf das Rückschlagventil 19 der in den Figuren 1 bis 4b gezeigten offenbarungsgemäßen Gassteuerungsvorrichtung 10. Dabei wird aus Richtung des einströmenden Gasstroms, in Inspirationsflussrichtung, auf das Rückschlagventil 19 geblickt. Gezeigt ist auch der Umgehungskanal 21, über welchen bei einer Blockade oder Störung des exspiratorischen Zweiges des Beatmungsgerätes der Gasstrom um das Rückschlagventil 19 herumführbar ist.

Dargestellt ist auch die Verschlusskappe 16 in Seitenansicht mit den Fortsätzen 18, mittels derer die Verschlusskappe 16 auf die Öffnung aufklippbar ist.

### Bezugszeichenliste

- 1: Beatmungsgerät
- 10: Gassteuerungsvorrichtung
- 11: erster Gaskanal
- 12: zweiter Gaskanal
- 13: Schalteinrichtung, Ventilblock
- 14: Öffnung
- 15: Dichtung, zweites Ventil
- 16: Verschlusskappe, zweites Ventil
- 17: Gewicht
- 18: Fortsatz der Verschlusskappe
- 19: erstes Ventil, Rückschlagventil
- 20: Anschluss
- 21: Umgehungskanal
- 22: Rand der Öffnung, Erhebung
- 23: Begrenzung der Dichtung
- 24: Rand des Umgehungskanals
- 27: Beatmungsschlauch
- 28: Schlauchstutzen
- 29: Umgebung
- 30: (Atem-)Gasquelle, Gebläse

## Patentansprüche

1. Gassteuerungsvorrichtung (10) für ein Beatmungsgerät (1), umfassend einen ersten Gaskanal (11) mit einem ersten Ventil (19), wobei der erste Gaskanal (11) und das erste Ventil (19) eingerichtet sind, einen unter Druck stehenden Gasstrom in einer ersten Richtung passieren zu lassen und einen unter Druck stehenden Gasstrom in einer zweiten Richtung zu sperren, wobei der Gasstrom der ersten Richtung Atemgas einer Inspiration ist und der Gasstrom der zweiten Richtung Atemgas einer Exspiration ist, wobei eine Öffnung (14) von dem ersten Gaskanal (11) abzweigt und zu einem Umgehungskanal (21) für das erste Ventil (19) führt, wobei ein zweites Ventil (15, 16) eingerichtet ist, einen Gasstrom von dem ersten Gaskanal (11) zu dem Umgehungskanal (21) zu sperren und/oder zumindest zeitweise zu ermöglichen, wobei eine Schalteinrichtung (13) eingerichtet ist, das zweite Ventil (15, 16) zu steuern, um einen Gasstrom von dem ersten Gaskanal (11) zu dem Umgehungskanal (21) zu sperren und/oder zumindest zeitweise zu ermöglichen, wobei eine Dichtung (15) eingerichtet ist, eine gasleitende Verbindung zwischen der Öffnung (14) und dem Umgehungskanal (21) zu verschließen oder zu öffnen, wobei die Dichtung (15) auf der Öffnung (14) aufliegend angeordnet ist und ein Gewicht (17) umfasst, das ringförmig in der Mitte der Dichtung (15) ausgebildet ist, wobei die Dichtung (15) aus einem elastomeren Material ausgebildet ist und das Gewicht (17) aus einem Metall ausgebildet ist.

2. Gassteuerungsvorrichtung (10) nach Anspruch 1,
wobei das erste Ventil (19) ein pneumatisch betätigbares Rückschlagventil (19) ist, das durch die Kraft des unter Druck stehenden Gasstroms in der zweiten Richtung betätigt werden kann, sodass der Gasstrom das Rückschlagventil (19) in der zweiten Richtung nicht passieren kann.

3. Gassteuerungsvorrichtung (10) nach Anspruch 2,
wobei eine Atemgasquelle (30) eingerichtet ist, Atemgas aus der Umgebung (29) anzusaugen und entlang des ersten Gaskanals (11) für die Inspiration in der ersten Richtung zu einem Schlauchstutzen (28) und einem Beatmungsschlauch (27) zu fördern, wobei der Gasstrom das geöffnete Rückschlagventil (19) passiert.

4. Gassteuerungsvorrichtung (10) nach einem der vorhergehenden Ansprüche,
wobei die Schalteinrichtung (13) eingerichtet ist, das zweite Ventil (15, 16) zu öffnen, sodass der unter Druck stehende Gasstrom der Exspiration durch die Öffnung (14) und den Umgehungskanal (21) zumindest teilweise in die Umgebung (29) gelangt.

5. Gassteuerungsvorrichtung (10) nach einem der vorhergehenden Ansprüche,
wobei die Dichtung (15) so eingerichtet ist, dass - wenn sie mit einem Steuerdruck beaufschlagt wird - sie sich dichtend an einen Rand (22) der Öffnung (14) anlegt und so einen Gasstrom von der Öffnung (14) in den Umgehungskanal (21) verhindert.

6. Gassteuerungsvorrichtung (10) nach einem der vorhergehenden Ansprüche,
wobei das zweite Ventil (15, 16) als pneumatisches Ventil ausgeführt ist, wobei ein zweiter Gaskanal (12) eingerichtet ist, einen pneumatischen Steuerdruck auf die Oberfläche der Dichtung (15) zu leiten.

7. Gassteuerungsvorrichtung (10) nach Anspruch 6,
wobei der zweite Gaskanal (12) mit einem Beatmungsschlauch (27) oder dem ersten Gaskanal (11) oder einem Gebläse (30) pneumatisch verbunden ist und die Schalteinrichtung (13) eingerichtet ist, die pneumatische Verbindung zumindest zeitweise zu öffnen oder zu verschließen.

8. Gassteuerungsvorrichtung (10) nach Anspruch 6 oder 7 rückbezogen auf Anspruch 2,
wobei die Schalteinrichtung (13) eingerichtet ist, einen Gasstrom oder Druck des zweiten Gaskanals (12) zu steuern und eine Vorbeiführung des Gasstroms des ersten Gaskanals (11) um das Rückschlagventil (19) durch die Öffnung (14) zu ermöglichen.

9. Gassteuerungsvorrichtung (10) nach einem der Ansprüche 6 bis 8,
wobei die Schalteinrichtung (13) eingerichtet ist, den zweiten Gaskanal (12) freizugeben oder zu sperren, und/oder wobei die Dichtung (15) ausgebildet ist, bei einem gesperrten zweiten Gaskanal (12) einen Durchfluss des Gasstroms des ersten Gaskanals (11) durch die Öffnung (14) zu ermöglichen.

10. Gassteuerungsvorrichtung (10) nach einem der Ansprüche 6 bis 9,
ferner umfassend eine Verschlusskappe (16), die so eingerichtet ist, dass sie auf die Öffnung (14) aufgesetzt werden kann, wobei die Verschlusskappe (16) einen mit dem zweiten Gaskanal (12) verbindbaren Anschluss (20) umfasst.

11. Gassteuerungsvorrichtung (10) nach einem der Ansprüche 6 bis 10,
wobei die Schalteinrichtung (13) eingerichtet ist, bei einem Normalbetrieb eines exspiratorischen Zweiges den zweiten Gaskanal (12) freizugeben und einen Gasstrom oder Druck über den zweiten Gaskanal (12) und einen Anschluss (20) auf die Dichtung (15) zu leiten und so die Öffnung (14) zu schließen und/oder bei einer Blockade oder Störung eines exspiratorischen Zweiges den zweiten Gaskanal (12) zu sperren und so den Umgehungskanal (21) für exspiratorisches Atemgas zu öffnen.

12. Gassteuerungsvorrichtung (10) nach einem der Ansprüche 6 bis 11,
wobei das zweite Ventil (15, 16) die Dichtung (15) umfasst, die eingerichtet ist, bei einem gesperrten zweiten Gaskanal (12) einem Gasstrom des ersten Gaskanals (11) einen Durchlass durch die Öffnung (14) zu ermöglichen.

13. Gassteuerungsvorrichtung (10) nach einem der vorhergehenden Ansprüche,
wobei die Dichtung (15) eine Ausprägung aufweist, die im Bereich des Gewichts (17) in Richtung des ersten Gaskanals (11) weisend ausgebildet ist.

14. Gassteuerungsvorrichtung (10) nach einem der vorhergehenden Ansprüche,
ferner umfassend einen Ventilblock (13) mit dem ersten Gaskanal (11), einem zweiten Gaskanal (12), dem ersten Ventil (19) und dem zweiten Ventil (15, 16), wobei der erste Gaskanal (11) das erste Ventil (19) und die Öffnung (14) aufweist, wobei dem zweiten Ventil (15, 16) zum Sperren und/oder Ermöglichen des Gasstroms von dem ersten Gaskanal (11) zu dem Umgehungskanal (21) der zweite Gaskanal (12) als Anschluss für einen Steuerdruck zugeordnet ist.

15. Beatmungsgerät (1), umfassend eine Gassteuerungsvorrichtung (10) nach einem der vorhergehenden Ansprüche.

## Claims

1. A gas control device (10) for a ventilator (1), comprising a first gas channel (11) with a first valve (19), wherein the first gas channel (11) and the first valve (19) are configured to let a pressurized gas stream pass in a first direction and to block a pressurized gas stream in a second direction, wherein the gas stream in the first direction is respiratory gas of an inspiration and the gas stream in the second direction is respiratory gas of an expiration, wherein an opening (14) branches off from the first gas channel (11) and leads to a bypass channel (21) for the first valve (19), wherein a second valve (15, 16) is configured to block and/or at least temporarily enable a gas stream from the first gas channel (11) to the bypass channel (21), wherein a switching apparatus (13) is configured to control the second valve (15, 16) in order to block and/or at least temporarily enable a gas stream from the first gas channel (11) to the bypass channel (21), wherein a seal (15) is configured to close or open a gas-conducting connection between the opening (14) and the bypass channel (21), wherein the seal (15) is arranged so as to rest on the opening (14) and comprises a weight (17), which is formed in the shape of a ring in the center of the seal (15), wherein the seal (15) is formed from an elastomer material and the weight (17) is formed from a metal.

2. The gas control device (10) according to claim 1,
wherein the first valve (19) is a pneumatically actuated check valve (19), which can be actuated by the force of the pressurized gas stream in the second direction so that the gas stream cannot pass the check valve (19) in the second direction.

3. The gas control device (10) according to claim 2,
wherein a respiratory gas source (30) is configured to suck in respiratory gas from the environment (29) and convey it along the first gas channel (11) for the inspiration in the first direction to a hose connector (28) and a ventilation hose (27), wherein the gas stream passes the open check valve (19).

4. The gas control device (10) according to one of the preceding claims,
wherein the switching apparatus (13) is configured to open the second valve (15, 16) so that at least some of the pressurized gas stream of the expiration passes through the opening (14) and the bypass channel (21) into the environment (29).

5. The gas control device (10) according to one of the preceding claims,
wherein the seal (15) is configured such that - when a control pressure is applied - it lies on an edge (22) of the opening (14) in a sealing manner and thus prevents a gas stream from flowing from the opening (14) into the bypass channel (21).

6. The gas control device (10) according to one of the preceding claims,
wherein the second valve (15, 16) is designed as a pneumatic valve, wherein a second gas channel (12) is configured to conduct a pneumatic control pressure onto the surface of the seal (15).

7. The gas control device (10) according to claim 6,
wherein the second gas channel (12) is pneumatically connected to a ventilation hose (27) or the first gas channel (11) or a fan (30), and the switching apparatus (13) is configured to at least temporarily open or close the pneumatic connection.

8. The gas control device (10) according to claim 6 or 7 related to claim 2,
wherein the switching apparatus (13) is configured to control a gas stream or pressure of the second gas channel (12) and to enable the gas stream of the first gas channel (11) to go past the check valve (19) and through the opening (14).

9. The gas control device (10) according to one of claims 6 to 8,
wherein the switching apparatus (13) is configured to release or block the second gas channel (12), and/or wherein the seal (15) is formed to enable the gas stream of the first gas channel (11) to flow through the opening (14) when a second gas channel (12) is blocked.

10. The gas control device (10) according to one of claims 6 to 9,
also comprising a closure cap (16), which is configured such that it can be placed onto the opening (14), wherein the closure cap (16) comprises a connection (20) that can be connected to the second gas channel (12).

11. The gas control device (10) according to one of claims 6 to 10,
wherein the switching apparatus (13) is configured, during normal operation of an expiratory branch, to release the second gas channel (12) and to conduct a gas stream or pressure onto the seal (15) via the second gas channel (12) and a connection (20) and thus to close the opening (14) and/or, when there is a blockage or malfunction of an expiratory branch, to block the second gas channel (12) and thus to open the bypass channel (21) for expiratory respiratory gas.

12. The gas control device (10) according to one of claims 6 to 11,
wherein the second valve (15, 16) comprises the seal (15), which is configured to enable a gas stream of the first gas channel (11) to pass through the opening (14) when a second gas channel (12) is blocked.

13. The gas control device (10) according to one of the preceding claims,
wherein the seal (15) has a bulge pointing in the direction of the gas channel (11) in the region of the weight (17).

14. The gas control device (10) according to one of the preceding claims,
also comprising a valve block (13) with the first gas channel (11), a second gas channel (12), the first valve (19), and the second valve (15, 16), wherein the first gas channel (11) has the first valve (19) and the opening (14), wherein the second gas channel (12), as a connection for a control pressure, is assigned to the second valve (15, 16) for blocking and/or enabling the gas stream from the first gas channel (11) to the bypass channel (21).

15. A ventilator (1), comprising a gas control device (10) according to one of the preceding claims.

## Revendications

1. Dispositif de commande de gaz (10) pour un appareil respiratoire (1), comportant un premier canal de gaz (11) avec une première soupape (19), dans lequel le premier canal de gaz (11) et la première soupape (19) sont configurés pour laisser passer un flux de gaz sous pression dans une première direction et pour bloquer un flux de gaz sous pression dans une deuxième direction, dans lequel le flux de gaz de la première direction est du gaz respiratoire d'une inspiration et le flux de gaz de la deuxième direction est du gaz respiratoire d'une expiration, dans lequel une ouverture (14) bifurque à partir du premier canal de gaz (11) et mène vers un canal de dérivation (21) pour la première soupape (19), dans lequel une deuxième soupape (15, 16) est configurée pour bloquer et/ou permettre au moins temporairement un flux de gaz allant du premier canal de gaz (11) vers le canal de dérivation (21), dans lequel un appareil de commutation (13) est configuré pour commander la deuxième soupape (15, 16) afin de bloquer et/ou permettre au moins temporairement un flux de gaz allant du premier canal de gaz (11) vers le canal de dérivation (21), dans lequel un moyen d'étanchéité (15) est configuré pour fermer ou ouvrir une liaison conductrice de gaz entre l'ouverture (14) et le canal de dérivation (21), dans lequel le moyen d'étanchéité (15) est disposé de manière à reposer sur l'ouverture (14) et comporte un poids (17), lequel est conçu de façon annulaire au centre du moyen d'étanchéité (15), dans lequel le moyen d'étanchéité (15) est constitué d'un matériau élastomère et le poids (17) est constitué d'un métal.

2. Dispositif de commande de gaz (10) selon la revendication 1,
dans lequel la première soupape (19) est une soupape antiretour (19) actionnable de façon pneumatique, laquelle peut être actionnée par la force du flux de gaz sous pression dans la deuxième direction, de sorte que le flux de gaz ne peut pas passer par la soupape antiretour (19) dans la deuxième direction.

3. Dispositif de commande de gaz (10) selon la revendication 2,
dans lequel une source de gaz respiratoire (30) est configurée pour aspirer du gaz respiratoire à partir de l'environnement (29) et pour le transporter le long du premier canal de gaz (11) pour l'inspiration dans la première direction vers un embout de tube (28) et un tube de ventilation (27), dans lequel le flux de gaz passe par la soupape antiretour (19) ouverte.

4. Dispositif de commande de gaz (10) selon l'une des revendications précédentes,
dans lequel l'appareil de commutation (13) est configuré pour ouvrir la deuxième soupape (15, 16) de manière à ce que le flux de gaz sous pression de l'expiration s'échappe au moins partiellement dans l'environnement (29) à travers l'ouverture (14) et le canal de dérivation (21).

5. Dispositif de commande de gaz (10) selon l'une des revendications précédentes,
dans lequel le moyen d'étanchéité (15) est configuré - lorsqu'il est sollicité avec une pression de commande - de manière à s'appliquer de façon étanche sur un bord (22) de l'ouverture (14) et empêche ainsi un flux de gaz à partir de l'ouverture (14) vers le canal de dérivation (21).

6. Dispositif de commande de gaz (10) selon l'une des revendications précédentes,
dans lequel la deuxième soupape (15, 16) est réalisée comme une soupape pneumatique, dans lequel un deuxième canal de gaz (12) est configuré pour guider une pression de commande pneumatique vers la surface du moyen d'étanchéité (15).

7. Dispositif de commande de gaz (10) selon la revendication 6,
dans lequel le deuxième canal de gaz (12) est relié pneumatiquement à un tube de ventilation (27) ou au premier canal de gaz (11) ou à une soufflerie (30) et l'appareil de commutation (13) est configuré pour ouvrir ou fermer au moins temporairement la liaison pneumatique.

8. Dispositif de commande de gaz (10) selon la revendication 6 ou 7 en liaison avec la revendication 2,
dans lequel l'appareil de commutation (13) est configuré pour commander un flux de gaz ou une pression du deuxième canal de gaz (12) et pour permettre un guidage du flux de gaz du premier canal de gaz (11) autour de la soupape antiretour (19) à travers l'ouverture (14).

9. Dispositif de commande de gaz (10) selon l'une des revendications 6 à 8,
dans lequel l'appareil de commutation (13) est configuré pour libérer ou bloquer le deuxième canal de gaz (12), et/ou dans lequel le moyen d'étanchéité (15) est conçu pour permettre un passage du flux de gaz du premier canal de gaz (11) par l'ouverture (14) lorsque le deuxième canal de gaz (12) est bloqué.

10. Dispositif de commande de gaz (10) selon l'une des revendications 6 à 9,
comportant en outre un capuchon de fermeture (16), lequel est configuré de manière à pouvoir être placé sur l'ouverture (14), dans lequel le capuchon de fermeture (16) comporte un raccord (20) apte à être relié au deuxième canal de gaz (12).

11. Dispositif de commande de gaz (10) selon l'une des revendications 6 à 10,
dans lequel l'appareil de commutation (13) est configuré pour libérer le deuxième canal de gaz (12) lors d'un fonctionnement normal d'une branche expiratoire et pour guider un flux de gaz ou une pression vers le moyen d'étanchéité (15) par le biais du deuxième canal de gaz (12) et d'un raccord (20) et pour ainsi fermer l'ouverture (14) et/ou pour bloquer le deuxième canal de gaz (12) lors d'une obstruction ou d'une panne d'une branche expiratoire et ainsi ouvrir le canal de dérivation (21) pour du gaz respiratoire expiratoire.

12. Dispositif de commande de gaz (10) selon l'une des revendications 6 à 11,
dans lequel la deuxième soupape (15, 16) comporte le moyen d'étanchéité (15), lequel est configuré pour permettre à un flux de gaz du premier canal de gaz (11) de passer à travers l'ouverture (14) lorsque le deuxième canal de gaz (12) est bloqué.

13. Dispositif de commande de gaz (10) selon l'une des revendications précédentes,
dans lequel le moyen d'étanchéité (15) présente un relief, lequel est conçu de manière à être orienté vers le premier canal de gaz (11) dans la région du poids (17).

14. Dispositif de commande de gaz (10) selon l'une des revendications précédentes,
comportant en outre un bloc de soupapes (13) avec le premier canal de gaz (11), un deuxième canal de gaz (12), la première soupape (19) et la deuxième soupape (15, 16), dans lequel le premier canal de gaz (11) présente la première soupape (19) et l'ouverture (14), dans lequel le deuxième canal de gaz (12) est attribué comme raccord pour une pression de commande à la deuxième soupape (15, 16) pour bloquer et/ou permettre le flux de gaz à partir du premier canal de gaz (11) vers le canal de dérivation (21).

15. Appareil respiratoire (1), comportant un dispositif de commande de gaz (10) selon l'une des revendications précédentes.
